# EUROPÄISCHE PATENTANMELDUNG

(11) **EP 1 361 471 A1**
(43) Veröffentlichungstag der Anmeldung: **12.11.2003**
(21) Anmeldenummer: 03008614.4
(22) Anmeldetag: 15.04.2003
(51) Int. Cl.: G02C 5/22

(54) **Brille, insbesondere Schutzbrille**

(30) Priorität: 07.05.2002 DE 20207180 U
(71) Anmelder: Uvex Arbeitsschutz GmbH, 90766 Fürth (DE)
(72) Erfinder: Brück, Stefan, 90419 Nürnberg (DE)
(74) Vertreter: Schneck, Herbert, Dipl.-Phys., Dr.

(57) **Zusammenfassung**

Bei einer Brille, insbesondere Schutzbrille, mit einer Scheibenanordnung (2) und in den äußeren Lagerbereichen (6) der Scheibenanordnung (2) angeordneten Lageranordnung (7) für Bügel (3), die für den Gebrauch aufschwenkbar und für die Aufbewahrung einschwenkbar sind, ist zur besonders einfachen und kostengünstigen Herstellung vorgesehen, dass auf jeder Seite je zwei voneinander beabstandete Ösen (16) angeordnet sind, dass jeder Bügel (3) zwei im Abstand voneinander verlaufende, elastisch nachgiebige Endteile (25) aufweist, und dass die Endteile (25) Lagerzapfen (34) aufweisen, die unter elastisch federndem Zusammendrücken der Endabschnitte (26) im ganz oder teilweise eingeschwenkten Zustand in die Ösen (16) einrastbar sind, wobei an jedem Endabschnitt (27) der Scheibenanordnung (2) zwischen den Endteilen (25) jedes Bügels (3) ein Abstandshalter (19) angeordnet ist, der im aufgeschwenkten Gebrauchszustand die Endteile (25) zueinander auf Abstand hält und die Lagerzapfen (34) in den Ösen (16) arretiert.

## Beschreibung

Die Erfindung richtet sich auf eine Brille, insbesondere Schutzbrille, mit einer Scheibenanordnung und in den äußeren Lagerbereichen der Scheibenanordnung angeordneten Lageranordnung für Bügel, die für den Gebrauch aufschwenkbar und für die Aufbewahrung einschwenkbar sind.

Herkömmlicherweise sind die Bügel einer Brille mit der Scheibenanordnung über Gelenk-Stifte schwenkbar verbunden. Nachteilig bei diesen bekannten Brillen ist, dass die Montage relativ zeitintensiv und somit kostenaufwendig ist. Dies ist vor allem darauf zurückzuführen, dass in Ausnehmungen, die sowohl seitlich der Scheibenanordnung als auch in den Endabschnitten der Bügel ausgebildet sind, Gelenk-Stifte zur Herstellung eines Schwenklagers einzuführen sind, wobei die Gelenk-Stifte anschließend in ihrer Position festgelegt werden müssen.

Hiervon ausgehend liegt der Erfindung die Aufgabe zugrunde, eine Brille der gattungsgemäßen Art derart auszugestalten, dass diese besonders einfach und kostengünstig hergestellt werden kann.

Diese Aufgabe wird erfindungsgemäß dadurch gelöst, dass auf jeder Seite je zwei voneinander beabstandete Ösen angeordnet sind, dass jeder Bügel zwei im Abstand voneinander verlaufende, elastisch nachgiebige Endteile aufweist, und dass die Endteile Lagerzapfen aufweisen, die unter elastisch federndem Zusammendrücken der Endabschnitte im ganz oder teilweise eingeschwenkten Zustand in die Ösen einrastbar sind, wobei an jedem Endabschnitt der Scheibenanordnung zwischen den Endteilen jedes Bügels ein Abstandshalter angeordnet ist, der im aufgeschwenkten Gebrauchszustand die Endteile zueinander auf Abstand hält und die Lagerzapfen in den Ösen arretiert. Die Verbindung der Bügel mit der Scheibenanordnung erfolgt durch Verrastung, was eine besonders schnelle und auch einfache Montage der Brille zufolge hat. Separate Verbindungselemente oder Montage-Hilfsmittel sind nicht erforderlich. Da die Abstandshalter die Lagerzapfen in den Ösen im Gebrauchszustand der Brille arretieren, wird außerdem eine besonders funktionssichere Verbindung der Bügel mit der Scheibenanordnung erzielt. Ein Lösen der Lagerzapfen aus den Ösen ist im Gebrauchszustand nicht möglich.

Vorzugsweise sind die Abstandshalter einstückig mit dem Rahmen gespritzt. Hierdurch wird einerseits eine besonders kostengünstige Fertigung der Brille erzielt, andererseits ist ein Lösen der Abstandshalter von dem Rahmen nicht möglich.

Es ist zweckmäßig, dass die Endteile der Bügel im aufgeschwenkten Gebrauchszustand formschlüssig in die Zwischenräume zwischen den Ösen und dem Abstandshalter eingreifen. Die Endteile können im Gebrauchszustand der Brille nicht verschwenkt und somit aus den Ösen gelöst werden. Im aufgeschwenkten Gebrauchszustand sind die Bügel unlösbar mit der Scheibenanordnung verbunden.

Gemäß einer vorteilhaften Ausgestaltung sind die Ösen an einstückig mit dem Rahmen ausgebildeten, auf den Benutzer zu vorspringenden Ansätzen ausgebildet. Diese einstückige Ausgestaltung hat wiederum eine besonders kostengünstige Fertigung der erfindungsgemäßen Brille zufolge. Da die Ansätze auf den Benutzer zu vorspringen, ist die Brille im Aufbewahrungszustand, d. h. mit eingeschwenkten Bügeln, besonders kompakt.

Eine weitere konstruktive Ausgestaltung zeichnet sich dadurch aus, dass die Brille dreiteilig, bestehend aus Scheibenanordnung und den beiden Bügeln, ausgebildet ist. Aufgrund dessen kann die erfindungsgemäße Brille besonders einfach und schnell montiert werden.

Nachfolgend wird die Erfindung anhand eines bevorzugten Ausführungsbeispiels in Verbindung mit der Zeichnung näher beschrieben. Dabei zeigen:
- Fig. 1: eine Aufsicht auf eine erfindungsgemäße Brille,
- Fig. 2: eine perspektivische Vorderansicht der in Fig. 1 dargestellten Brille,
- Fig. 3: eine Seitenansicht der in Fig. 1 dargestellten Brille,
- Fig. 4: eine vergrößerte, perspektivische Ansicht einer Lageranordnung für einen Bügel der in Fig. 1 dargestellten Brille, wobei der teilweise dargestellte Bügel aufgeschwenkt ist, und
- Fig. 5: eine vergrößerte, perspektivische Ansicht einer Lageranordnung für einen Bügel der in Fig. 1 dargestellten Brille, wobei der teilweise dargestellte Bügel eingeschwenkt ist.

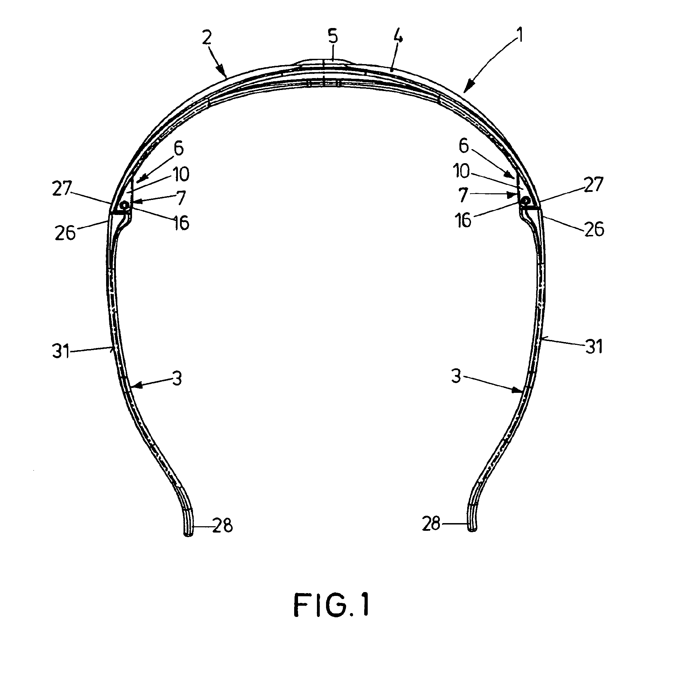

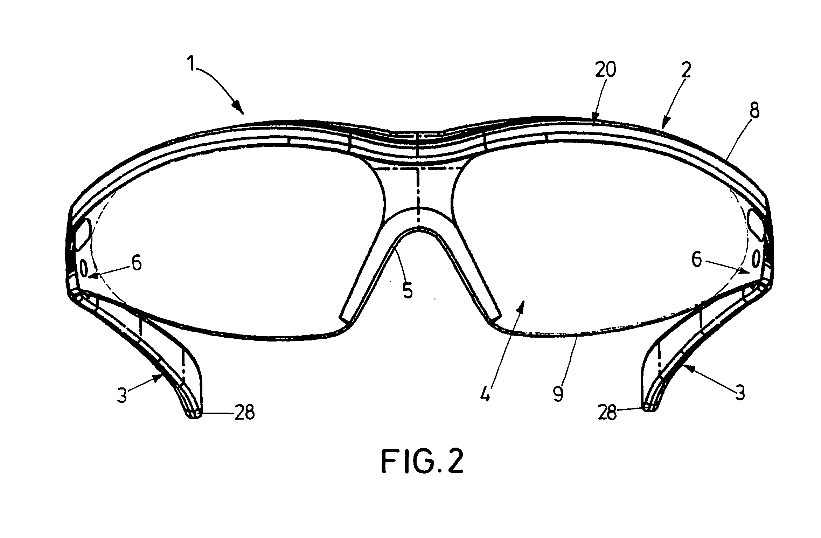

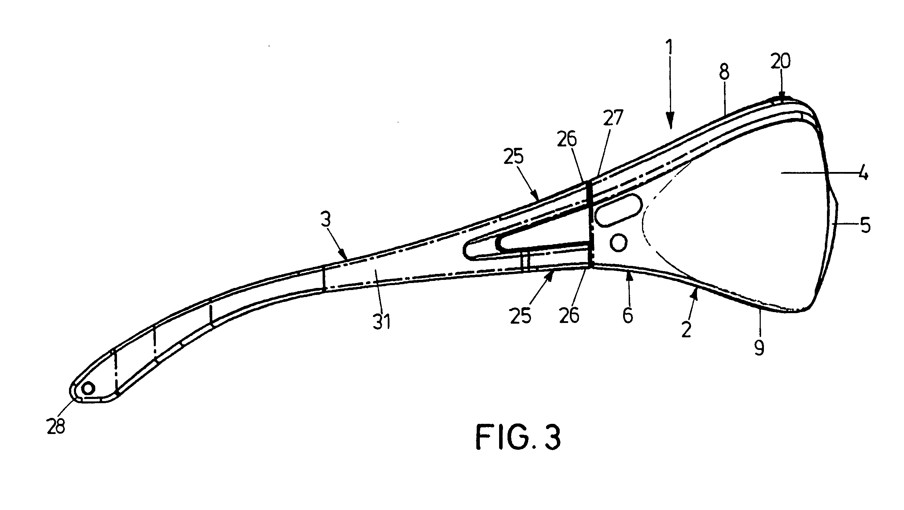

Eine in den Fig. 1 bis 3 dargestellte Schutzbrille 1 umfasst eine Scheibenanordnung 2 und Bügel 3, die seitlich an der Scheibenanordnung 2 angelenkt sind. Sowohl die Scheibenanordnung 2 als auch die Bügel 3 sind jeweils einstückig aus Kunststoff gespritzt.

Die Scheibenanordnung 2 umfasst einen Rahmen 20, eine Sicht-Scheibe 4, ein den Bereich der Nasenwurzel eines Brillenträgers überbrückendes Nasenbrückenteil 5 und zwei voneinander beabstandet angeordnete, äußere Lagerbereiche 6, die jeweils seitlich der Sicht-Scheibe 4 vorgesehen sind. Der Rahmen 20 verläuft oberhalb und seitlich der Sicht-Scheibe 4.

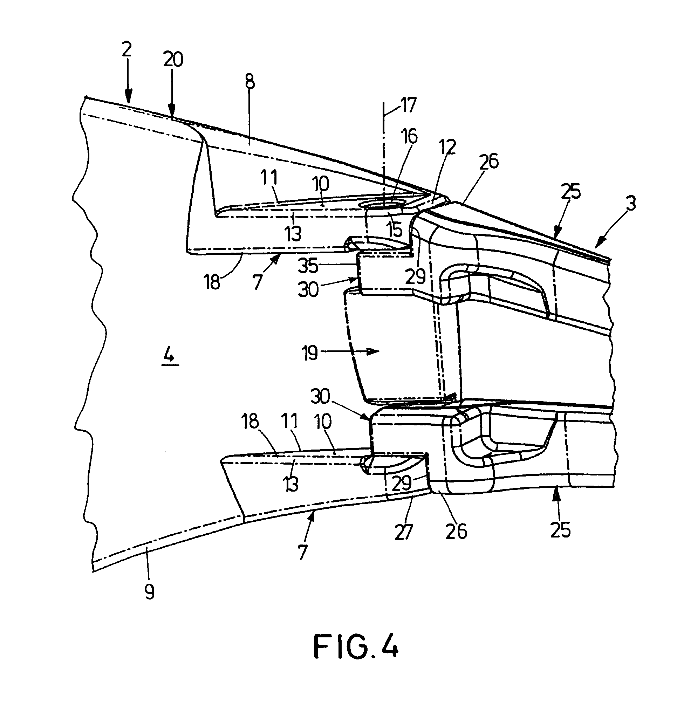

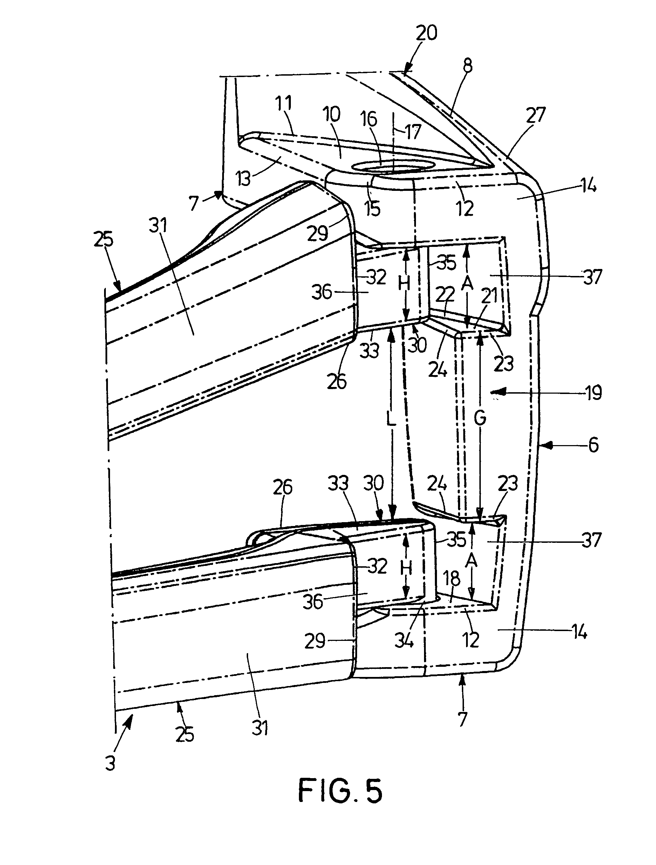

In jedem Lagerbereich 6 sind zwei voneinander beabstandet angeordnete Ansätze 7 ausgebildet, die auf einen Brillenträger (nicht dargestellt) vorspringen. Wie aus den Fig. 4 und 5 ersichtlich ist, verlaufen die Ansätze 7 im wesentlichen parallel zueinander und sind jeweils im Bereich einer oberen Abschlusskante 8 bzw. einer unteren Abschlusskante 9 der Scheibenanordnung 2 angeordnet. Die Ansätze 7 weisen im wesentlichen eine dreieckige Grundfläche 10 auf, die durch Kanten 11, 12 und 13 begrenzt sind. Die an den Rahmen 20 angrenzenden Kanten 11 sind an den Krümmungsradius des Rahmens 20 in diesem Bereich angepasst. Die Kanten 12 verlaufend fluchtend mit der jeweiligen Abschlussfläche 14 der Scheibenanordnung 2. Die Kanten 13 verlaufen in etwa senkrecht zu den Kanten 12, wobei der Übergang 15 zwischen den Kanten 12, 13 gekrümmt ausgebildet ist.

Die Ansätze 7 sind jeweils von einer, im Querschnitt kreisförmig ausgebildeten Öse 16 durchdrungen. Die Ösen 16 weisen jeweils eine Mittel-Längs-Achse 17 auf. Die Mittel-Längs-Achsen 17 der in einem Lagerbereich 6 vorgesehenen Ösen 16 verlaufen koaxial. Im Bereich der Ösen 16 sind die einander zugewandten Flächen 18 der Ansätze 7 eben ausgebildet und verlaufen parallel zueinander.

Zwischen den in einem Lagerbereich 6 ausgebildeten Ansätzen 7 ist jeweils ein Abstandshalter 19 mit dem Rahmen 20 der Schutzbrille 1 gespritzt. Zwischen einem Abstandshalter 19 und den benachbarten angeordneten Ansätzen 7 ist jeweils ein Abstand A vorgesehen. Es liegen somit jeweils zwei Zwischenräume 37 zwischen einem Abstandshalter 19 und den benachbart angeordneten Ansätzen 7 vor.

Die Grundfläche 21 der Abstandshalter 19 ist jeweils im wesentlichen dreieckförmig ausgebildet und verläuft parallel zu den Flächen 18 der Ansätze 7. Die Grundfläche 21 der Abstandshalter 7 weist drei Kanten 22, 23, 24 auf. Die an den Rahmen 20 angrenzenden Kanten 22 sind kürzer als die Kanten 11 der Ansätze 7 ausgebildet und sind an den Krümmungsradius des Rahmens 20 in diesem Bereich angepasst. Die hinteren Kanten 23 verlaufen fluchtend mit der Abschlussfläche 14 und sind kürzer als die Kanten 12 der Ansätze 7 ausgebildet. Die Kanten 23 und 24 verlaufen annähernd senkrecht zueinander. Die Länge der Kanten 23 ist so gewählt, dass die Abstandshalter 19 nicht in die imaginäre Verbindung zwischen den jeweiligen Ösen 16 eines Lagerbereiches 6 ragen. Die Abstandshalter 19 sind derart an dem Rahmen 20 angeordnet, dass sie auf einen Brillenträger zu vorspringen.

Die an der Scheibenanordnung 2 angelenkten Bügel 3 weisen jeweils zwei im Abstand voneinander verlaufende, elastisch nachgiebige Endteile 25 auf, deren Endabschnitte 26 an die Endabschnitte 27 der Scheibenanordnung 2 angrenzen. Die Endteile 25 laufen gabelartig zum Bügelende 28 aufeinander zu und weisen endseitig jeweils einen nach innen, über einen Absatz 29 abgesetzten Lagerkörper 30 auf. Die Lagerkörper 30 sind außerdem jeweils gegenüber der Außenfläche 31 der Bügel 3 über einen Absatz 32 nach innen versetzt. Die Absätze 29, 32 liegen in einer Ebene. Die Lagerkörper 30 weisen eine im wesentlichen trapezförmige Grundfläche 33 auf. Sämtliche an einem Bügel 3 ausgebildete Grundflächen 33 der Lagerkörper 30 erstrecken sich parallel zueinander. Die Grundflächen 33 verlaufen außerdem parallel zu den Grundflächen 21 der Abstandshalter 19 sowie zu den den Abstandshaltern 19 zugewandten Flächen 18 der Ansätze 7. Der Abstand L der einander zugewandten Grundflächen 33 entspricht in etwa dem Abstand G der Grundflächen 21 eines Abstandshalters 19 zueinander. Die Lagerkörper 30 weisen jeweils eine Höhe H auf, die im etwa dem Abstand A zwischen einem Ansatz 7 und einem Abstandshalter 19 entspricht.

Mit jedem Lagerkörper 30 ist ein senkrecht abstehender, im Querschnitt kreisförmig ausgebildeter Lagerzapfen 34 einstückig verbunden, wobei die Lagerzapfen 34 jeweils auf den einander abgewandten Grundflächen 33 der Lagerkörper 30 angeordnet sind. Die Lagerzapfen 34 weisen jeweils eine Mittel-Längs-Achse auf, die bei den an einem Bügel 3 angeordneten Lagerzapfen 34 koaxial zueinander verlaufen. Der Durchmesser eines Lagerzapfens 34 entspricht in etwa dem Durchmesser einer Öse 16. Die Lagerzapfen 34 sind derart angeordnet, dass ein Bereich ihrer Außenfläche mit den Abschlussflächen 35 der Lagerkörper 30 fluchtet. Die Abschlussflächen 35 verlaufen parallel zu den Absätzen 29 und 32. Die Höhe der Lagerzapfen 34 ist derart gewählt, dass im montierten Zustand die Lagerzapfen 34 nicht aus den Flächen 10 der Scheibenanordnung 2 herausragen. Die zwischen den Abschlussflächen 35 und den Absätzen 32 verlaufenden Seitenflächen 36 der Lagerkörper 30 sind gegenüber der Außenfläche 31 im Bereich des Endabschnitts 26 der Bügel 3 geneigt. Die Lagerzapfen 34 sind in den Ösen 16 schwenkbar gelagert. Die Ansätze 7 bilden eine Lageranordnung für die Bügel 3.

Um die Bügel 3 in ihren End-Stellungen festzulegen, ist jeweils im Bereich eines Lagerzapfens 34 eine geringfügige Erhebung (nicht dargestellt) an die Absätze 29 angespritzt.

Im aufgeschwenkten Gebrauchszustand greifen die Lagerkörper 30 formschlüssig in die Zwischenräume 37. Die Endteile 25 sind durch die Abstandshalter 19 zueinander auf Abstand gehalten, wodurch die Lagerzapfen 34 in den Ösen 16 arretiert sind. Ein Verschwenken der Endteile 25 ist im Gebrauchszustand nicht möglich.

In zumindest teilweise eingeschwenktem Zustand greifen die Lagerkörper 30 aufgrund ihrer Formgebung und der Ausgestaltung der Abstandshalter 19 nicht in die Zwischenräume 37 ein, was zur Folge hat, dass die Endteile 25 aufeinander zu geschwenkt werden können, wenn auf diese eine entsprechende Kraft ausgeübt wird. Im zusammengedrückten Zustand der Endteile 25 können die Lagerzapfen 34 aus den Ösen 16 geführt werden, sodass die Bügel 3 von der Scheibenanordnung 2 entfernt werden können. Umgekehrt können die Lagerzapfen 34 nur in einer eingeschwenkten Stellung der Bügel 3 und bei zusammengedrückten Endteilen 25 in die Ösen 16 eingeführt werden. Die Endteile 25 sind derart federnd ausgebildet, dass die Lagerzapfen 34 auch im eingeschwenkten Zustand funktionssicher in den Ösen 16 gelagert sind.

## Patentansprüche

1. Brille, insbesondere Schutzbrille, mit einer Scheibenanordnung (2) und in den äußeren Lagerbereichen (6) der Scheibenanordnung (2) angeordneten Lageranordnung (7) für Bügel (3), die für den Gebrauch aufschwenkbar und für die Aufbewahrung einschwenkbar sind, **dadurch gekennzeichnet, dass** auf jeder Seite je zwei voneinander beabstandete Ösen (16) angeordnet sind, dass jeder Bügel (3) zwei im Abstand voneinander verlaufende, elastisch nachgiebige Endteile (25) aufweist, und dass die Endteile (25) Lagerzapfen (34) aufweisen, die unter elastisch federndem Zusammendrücken der Endabschnitte (26) im ganz oder teilweise eingeschwenkten Zustand in die Ösen (16) einrastbar sind, wobei an jedem Endabschnitt (27) der Scheibenanordnung (2) zwischen den Endteilen (25) jedes Bügels (3) ein Abstandshalter (19) angeordnet ist, der im aufgeschwenkten Gebrauchszustand die Endteile (25) zueinander auf Abstand hält und die Lagerzapfen (34) in den Ösen (16) arretiert.

2. Brille nach Anspruch 1, **dadurch gekennzeichnet, dass** die Abstandshalter (19) einstückig mit dem Rahmen (20) gespritzt sind.

3. Brille nach Anspruch 1, **dadurch gekennzeichnet, dass** die Endteile (25) zum Bügelende (28) hin gabelartig aufeinander zulaufen.

4. Brille nach Anspruch 1, **dadurch gekennzeichnet, dass** die Endteile (25) der Bügel (3) im aufgeschwenkten Gebrauchszustand formschlüssig in die Zwischenräume (37) zwischen den Ösen (16) und dem Abstandshalter (19) eingreifen.

5. Brille nach Anspruch 1, **dadurch gekennzeichnet, dass** die Ösen (16) an einstückig mit dem Rahmen (20) ausgebildeten, auf den Brillenträger zu vorspringenden Ansätzen (7) ausgebildet sind.

6. Brille nach Anspruch 1, **dadurch gekennzeichnet, dass** die Brille (1) dreiteilig bestehend aus der Scheibenanordnung (2) und den beiden Bügeln (3) ausgebildet ist.
